# EUROPÄISCHE PATENTSCHRIFT

(11) **EP 1 159 938 B1**
(45) Veröffentlichungstag und Bekanntmachung des Hinweises auf die Patenterteilung: **29.10.2003**
(21) Anmeldenummer: 01810421.6
(22) Anmeldetag: 30.04.2001
(51) Int. Cl.: A61F 2/38

(54) **Knieprothese**
Knee prosthesis
Prothèse de genou

(30) Priorität: 29.05.2000 EP 00810467
(43) Veröffentlichungstag der Anmeldung: 05.12.2001
(73) Patentinhaber: Centerpulse Orthopedics Ltd., 6340 Baar (CH)
(72) Erfinder: Leclercq, Vincent, 8406 Winterthur (CH); Gyssler, Bernhard, 8810 Horgen (CH)
(74) Vertreter: Manitz, Finsterwald & Partner Gbr

(56) Entgegenhaltungen:
- EP-A- 0 634 156
- GB-A- 2 278 782
- US-A- 5 395 401
- US-A- 5 413 604
- US-A- 5 871 543
- US-A- 6 013 103

## Beschreibung

Die Erfindung handelt von einer Knieprothese nach dem Oberbegriff des Anspruchs 1.

In der Patentschrift U.S. 5,219,362 ist eine Knieprothese gezeigt, bei der auf der medialen Seite eine grosse Kontaktfläche zwischen der medialen Kondyle und einer gegenüberliegenden Tibiaplattform besteht. Bei der lateralen Kondyle besteht während der Flexion höchstens eine Linienberührung, was zu unerwünscht hohen Flächenpressungen führt.

US 6,013,103, die zur Bildung des Oberbegriffs des Anspruchs 1 herangezogen worden ist, enthält eine Aufzählung von Knieprothesen, bei denen die mediale Femurkondyle über einen grossen Artikulationsbereich Flächenkontakt mit ihrer Gegenfläche auf einer Tibiaplattform hat. Die laterale Femurkondyle ist ebenfalls mit einer Gegenfläche auf der Tibiaplattform in Eingriff. Es sind verschiedene Möglichkeiten aufgezählt, mit denen eine Schwenkung der Tibia relativ zu den Femurkondylen erreicht wird. Eine Möglichkeit besteht darin, für die ganze Auflage auf der Tibiaplattform eine Schwenkung zuzulassen, die durch äussere Begrenzungen, ähnlich wie Leitplanken, begrenzte Schwenkmöglichkeiten bietet. Eine andere Möglichkeit besteht darin, eine Schwenkbewegung der ganzen Auflage durch eine gekrümmte Führung zwangsläufig zu führen. Eine weitere Möglichkeit besteht darin, die Auflage zu teilen, um die Gegenfläche der lateralen Kondyle entlang der Auflage für die Gegenfläche der medialen Kondyle schwenkbar zu machen. Die hier gezeigten Konzepte setzen eine grosse Teilevielfalt voraus.

Aufgabe der Erfindung ist es, diesen Zustand zu verbessern. Dies wird durch die im kennzeichnenden Teil des Anspruchs 1 angegebenen Merkmale erreicht.

Diese Anordnung verhindert grosse Flächenpressungen und gestattet der Tibia während der Flexion entsprechend den angreifenden Bändern eine Drehbewegung der Tibiaplattform um einen Punkt M₂ auf ihrer medialen Gegenfläche A und mit der Drehbewegung eine Bewegung der lateralen Seite der Tibiaplattform gegen anterior, während das Meniskusteil der lateralen Seite relativ zur lateralen Kondyle stehen bleibt.

Die Verwendung von Kunststoffkörpern medial und lateral sowie der Umstand, dass die Tibiaplattform bezüglich ihrer Sagittalebene symmetrisch ausgeführt ist, hat den Vorteil, dass die Tibiaplattform wahlweise für ein linkes oder rechtes Knie einsetzbar ist, indem der mediale Kunststoffkörper fest verankerbar ist und das laterale Meniskusteil mit seinem Punkt M₂ auf einem Radius R₃ um den Punkt M₁ der medialen Seite schwenkbar ist.

Die abhängigen Ansprüche 2 bis 10 sind vorteilhafte Weiterbildungen der Erfindung. So ist die Tibiaplattform aus einer Mittelstellung die der Extensionsstellung entspricht und in der die Punkte M₁, M₁', M₂, M₂' in einer Transversalebene der Tibiaplattform liegen, um einen Winkel α₁ ≥ 0 gegen posterior in eine Stellung schwenkbar, die der Hyperextension entspricht und bei vollständiger Flexion um einen Winkel α₂ zwischen 5° und 20° gegen anterior schwenkbar. Weiterhin kann es von Vorteil sein, die Summe der Winkel α₁ und α₂ auf einen Wert zwischen 12° und 18° zu beschränken, um konstruktiv eine grössere Gegenfläche B für die laterale Kondyle zu erreichen.

Ein kreisbogenförmiger Wulst der Tibiaplattform, der über eine Gleitebene für das Meniskusteil vorsteht und in eine Nut des Meniskusteils zur Führung um den ersten Punkt M₁ eingreift hat den Vorteil, dass sich keine Fremdkörper auf der Gleitebene oder in der Nut sammeln können und gequetscht werden können, da sie durch die Relativbewegung zwischen Meniskusteil und Tibiaplattform immer wieder abgestreift werden. Dies setzt voraus, dass in der Bewegungsrichtung des Meniskusteils keine grösseren Schultern, sondern höchstens Endanschläge für die Bewegung über die Gleitebene vorstehen.

Die Tibiaplattform kann aber auch selbst auf der lateralen Seite mit einer breiten kreisförmigen Nut versehen sein, deren Begrenzungsradien den gemeinsamen Mittelpunkt M₁ auf der medialen Seite haben, und das Meniskusteil ist als Teil eines Kreisringes ausgeführt, der durch die Nut geführt ist.

Durch die kugelförmige Kontaktfläche auf der medialen Seite und durch die kugelförmige und auf der Unterseite ebene Kontaktfläche des lateralen Meniskusteils kann die Flächenpressung soweit gesenkt werden, dass medial auf der Tibiaplattform ein Kunststoffkörper eingesetzt ist und dass als Meniskusteil ein Kunststoffkörper eingesetzt ist; beide beispielsweise aus hochmolekularen Polyethylen.

Im folgenden wird die Erfindung anhand von Ausführungsbeispielen beschrieben. Es zeigen:
- Fig. 1: Schematisch eine Draufsicht auf eine Tibiaplattform eines rechten Knies mit einem fest verankerten Kunststoffteil auf der medialen Seite und einem beweglichen Meniskusteil aus Kunststoff auf der lateralen Seite;
- Fig. 2: schematisch einen Schnitt der Figur 1 in einer Transversalebene, in der auch die Kondylen des Kniegelenks angedeutet sind.
- Fig. 3: schematisch ohne Kunststoffkörper eine Draufsicht auf die Tibiaplattform von Figur 1 mit zur Sagittalebene symmetrischen Vertiefungen und Führungswulsten;
- Fig. 4: schematisch eine Draufsicht auf eine symmetrische Tibiaplattform, die mit Kunststoffkörpern für ein rechts Knie bestückt ist; und
- Fig. 5: schematisch einen Schnitt der Figur 4.

Die Figuren zeigen eine Knieprothese mit einer medialen Kondyle 1 und einer lateralen Kondyle 5 deren Laufflächen 2 und deren auf einer Tibiaplattform 3 gelegenen Gegenflächen A, B Ausschnitte von Kugelflächen mit Radien R₁, R₂ sind, wobei deren Mittelpunkt M₁' und M₂' in ihrer vertikalen Projektion auf die Gegenflächen A, B die Punkte M₁ und M₂ bilden. Die laterale Gegenfläche B gehört zu einem lateralen Meniskusteil 7 das auf einer Ebene 13 der Tibiaplattform mit dem Punkt M₂ auf einem Kreisbogen mit Radius R₃ um den Punkt M₁ geführt schwenkbar ist, wobei der Radius R₃ einem Abstand a der Mittelpunkte M₁', M₂' entspricht.

Im folgenden werden gleiche Hinweiszeichen für gleiche Funktionen verwendet.

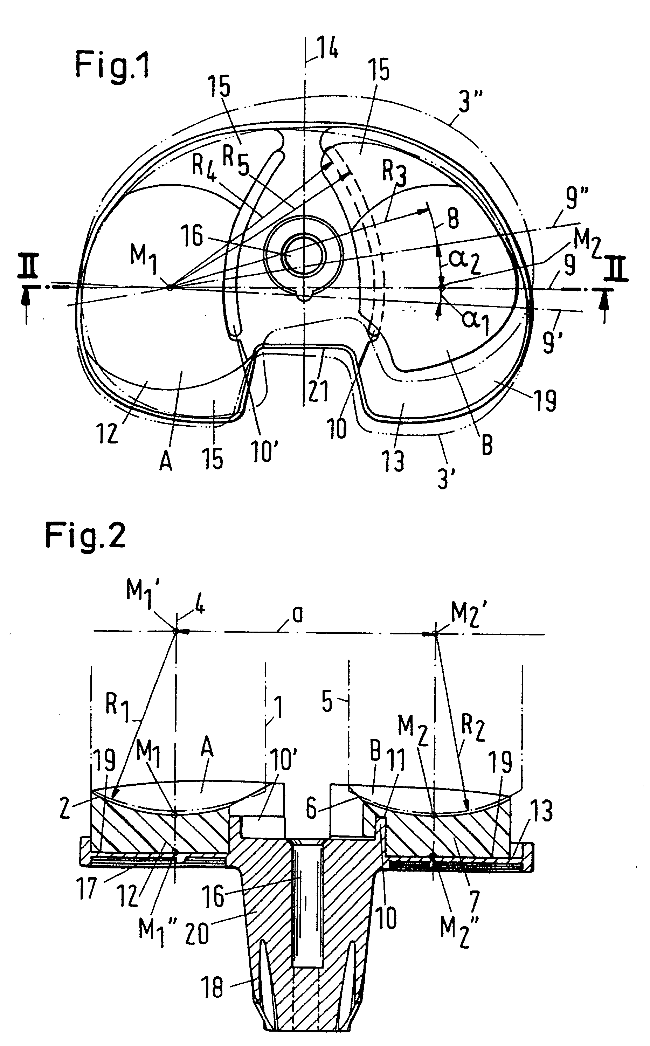

Im Beispiel der Figuren 1, 2 und 3 ist die Tibiaplattform 3 als zu ihrer sagittalen Mittelebene 14 symmetrisch ausgeführt, besitzt gegen distal einen Zapfen 20 mit Rippen 18 und zentraler Bohrung 16 und ist auf ihrer Oberseite medial und lateral jeweils mit einer ebenen Vertiefung 19 versehen. Zur Mitte hin ist die Vertiefung jeweils durch einen kreisförmigen Wulst 10, 10' mit begrenzenden Radien R₄ und R₅ abgeschlossen. Posterior besteht eine Ausnehmung 21 für das hintere Kreuzband. Auf der Unterseite ist eine Beschichtung 17 zur besseren Verankerung in Knochen oder in Knochenzement angebracht. Diese Beschichtung können beispielsweise Metallgitter sein.

In Figur 2 sind zwei Kondylen 1, 5 der Femurseite mit Phantomlinien angedeutet. Die Kondylen 1, 5 und ihre Gegenflächen A, B auf der Tibiaplattform 3 sind Ausschnitte von Kugelflächen mit Radien R₁, R₂ und Mittelpunkten M₁', M₂'. Die Kondylen drehen während der Flexion um eine Verbindungsgerade der Mittelpunkte M₁', M₂', welche zueinander einen Abstand "a" aufweisen. In der medialen Vertiefung 19 ist ein Kunststoffkörper 12 fixiert, der die Vertiefung 19 vollständig ausfüllt. In der lateralen Vertiefung 19 ist ein künstliches Meniskusteil 7 aus Kunststoff eingelegt, welches auf der Ebene 13 gleiten kann und mit einer Nut 11 am lateralen Wulst 10 geführt ist. Mit einer vertikalen Projektion der Mittelpunkte M₁' und M₂' entstehen an den Gegenflächen die Punkte M₁, M₂ und an dem Tibiaplateau 3 auf den Ebenen 3 die Punkte M₁" und M₂". Die Lauffläche 2 der medialen Kondyle 1 und die Lauffläche 6 der lateralen Kondyle 5 stehen seitlich etwas über. Das Meniskusteil 7 ist um den Punkt M₁ und um eine vertikale Gerade M₁', M₁ schwenkbar.

In Figur 1 ist die Tibiaplattform 3 in einer Extensionsstellung der (nicht gezeigten) Kondylen dargestellt, in der die Punkte M₁ und M₂ in einer Transversalebene 9 des Tibiaplateaus 3 liegen. Die Kondylen 1, 5 liegen jeweils auf den Gegenflächen A, B auf. Aus dieser Mittelstellung können die Kondylen in eine Hyperextensionsstellung weiterdrehen. Gleichzeitig erzeugen Bänder an der Tibia ein Moment, welches die Tibiaplattform 3 relativ zu den stillstehenden Punkten M₁ und M₂ um einen Winkel α₁ in Richtung posterior um den Punkt M₁ drehen. Diese verschobene Lage ist mit Phantomlinien durch eine Tibiaplattform 3' und ihre geschwenkte Transversalebene 9' dargestellt. Bei vollständiger Flexion der Kondylen 1, 5 greift ein Moment in Gegenrichtung an der Tibia an und erzeugt eine Drehbewegung der Tibiaplattform 3 um einen Winkel α₂ um den Drehpunkt M₁. In Phantomlinien ist eine entsprechend gegen anterior geschwenkte Tibiaplattform 3" mit ihrer geschwenkten Transversalebene 9" gezeigt. Der mediale Kunststoffkörper 12 und das laterale Meniskusteil weisen an ihrer Oberseite Abflachungen 15 auf, die verhindern, dass unnötig hohe Kugelkalotten entstehen.

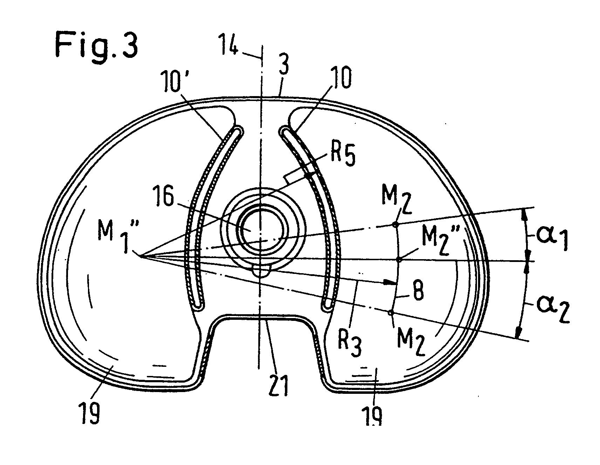

In Figur 3 ist dargestellt, welche Bewegungen der Punkt M₂ des Meniskusteils relativ zur Tibiaplattform 3 machen sollte, um die oben beschriebene Funktion auszufüllen. Der Radius R₅ für den Wulst 10 hat einen Betrag zwischen 25 und 50 mm; beispielsweise 33 mm. Die Summe der Winkel α₁ + α₂ liegt zwischen 12° und 18°; beispielsweise bei 15°.

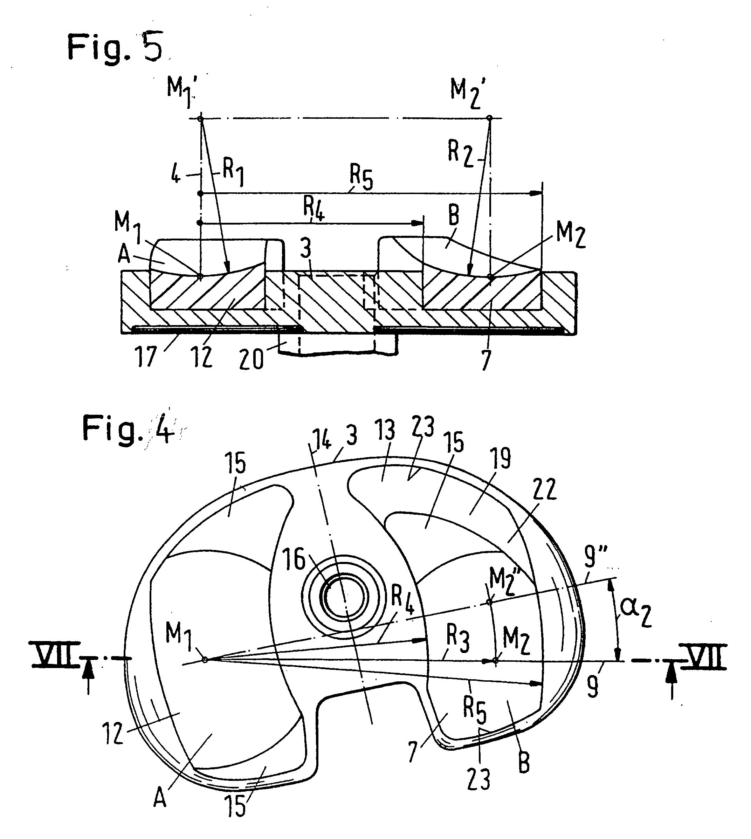

Eine weitere konstruktive Ausführung ist im Beispiel von Figur 4 und 5 gezeigt, statt kreisförmigen Führungswulsten haben symmetrische mediale und laterale Vertiefungen 19 in Form einer kreisbogenförmigen Nut 22 mit seitlich kreisförmigen Begrenzungen durch Radien R₄ und R₅, in denen ein mediales Kunststoffteil 12 fest verankert ist, während entlang den kreisförmigen Begrenzungen ein laterales Meniskusteil 7 mit seinem Punkt M₂ auf einem Radius R₃ relativ zur Tibiaplattform, um den Punkt M₁ schwenkbar ist. In Figur 4 ist eine Flexionsstellung angenommen, in der das laterale Meniskusteil 7 während der Flexion unverändert durch seine Kondyle an Ort gehalten wird, während die Tibiaplattform 3 mit ihrer Transversalebene 9 aus einer ursprünglichen Stellung um einen Winkel α₂ in eine Transversalebene 9" gegen anterior geschwenkt ist. Anschläge 23 begrenzen die Schwenkbewegung der Tibiaplattform 3 relativ zum lateralen Meniskusteil 7, welches in Drehrichtung kürzer als die Vertiefung 19 ist.

## Patentansprüche

1. Knieprothese mit einer medialen Kondyle (1), deren Lauffläche (2) und deren auf einer Tibiaplattform (3) gelegene Gegenfläche (A) Ausschnitte von Kugelflächen mit einem ersten Radius (R₁) sind, dessen Mittelpunkt (M₁') in seiner vertikalen Projektion mit einem ersten Punkt (M₁) auf der Gegenfläche (A) eine Achse (4) bildet, um welche eine laterale Kondyle (5) schwenkbar ist, wobei die laterale Kondyle (5) mit ihrer Lauffläche (6) und deren Gegenfläche (B) ebenfalls Ausschnitte von Kugelflächen mit einem zweiten Radius (R₂) mit Mittelpunkt (M₂') sind, dessen vertikale Projektion auf die Gegenfläche (B) einem zweiten Punkt (M₂) entspricht, und wobei die Gegenfläche (B) zu einem künstlichen, auf einer Ebene der Tibiaplattform verschiebbaren Meniskusteil (7) gehört, welches auf einem Kreisbogen geführt mit dem zweiten Punkt (M₂) um den ersten Punkt (M₁) in einem Abstand (R₃) schwenkbar ist, der dem Abstand (a) der Mittelpunkte (M₁', M₂') entspricht,
**dadurch gekennzeichnet, dass** die Tibiaplattform (3) bezüglich ihrer sagittalen Mittelebene (14) derart symmetrisch ausgeführt ist, dass sich jede Seite wahlweise zur Aufnahme eines fest verankerbaren Kunststoffkörpers (12) oder eines beweglichen Meniskusteils (7) eignet, um mit einer Tibiaplattform (3) rechte und linke Tibiagelenkhälften zu erstellen.

2. Knieprothese nach Anspruch 1, **dadurch gekennzeichnet, dass** bei festgehaltenem Meniskusteil die Tibiaplattform (3) aus einer Mittelstellung heraus, in der die Punkte (M₁, M₁'; M₂, M₂') in einer Transversalebene (9) der Tibiaplattform (3) liegen, um die Achse (4 = M₁, M₁') mit einem Winkel α₁ ≥ 0 gegen posterior und mit einem Winkel α₂ > α₁ gegen anterior schwenkbar ist.

3. Knieprothese nach Anspruch 2, **dadurch gekennzeichnet, dass** der Winkel α₂ zwischen 5° und 20° liegt.

4. Knieprothese nach einem der Ansprüche 1 bis 3, **dadurch gekennzeichnet, dass** die Führung des Meniskusteils (7) durch einen kreisbogenförmigen Wulst (10) der Tibiaplattform (3) auf der inneren Seite der lateralen Hälfte der Tibiaplattform (3) erfolgt, der in eine Nut (11) des Meniskusteils (7) eingreift.

5. Knieprothese nach einem der Ansprüche 1 bis 4, **dadurch gekennzeichnet, dass** die Gegenfläche (A) aus einem auf der Tibiaplattform (3) befestigten Kunststoffkörper (12) gebildet ist und dass das Meniskusteil (7) aus Kunststoff besteht.

6. Knieprothese nach einem der Ansprüche 1 bis 5, **dadurch gekennzeichnet, dass** ein ersten bzw. zweiden Radius (R₁, R₂) einer Kondyle (1, 5) um einen bestimmten Betrag kleiner als der zweiden bzw. ersten Radius (R₂, R₁) der anderen Kondyle (5, 1) ist, und dass die zu dieser kleineren Kondyle zugehörige Gegenfläche (A, B) um diesen bestimmten Betrag weiter nach oben vorsteht.

7. Knieprothese nach Anspruch 4, **dadurch gekennzeichnet, dass** der Wulst (10) mit seiner äusseren Führungsfläche einem Radius (R₅) zum ersten Punkt M₁ entspricht und dass der Radius (R₅) einem Betrag zwischen 25 mm und 50 mm entspricht.

8. Knieprothese nach einem der Ansprüche 2 bis 7, **dadurch gekennzeichnet, dass** die Summe Winkel α₁ und α₂ zwischen 12° und 18° liegt.

9. Knieprothese nach einem der Ansprüche 1 bis 3, **dadurch gekennzeichnet, dass** die Führung des Meniskusteils (7) durch eine kreisbogenförmige Nut (22) mit einem Aussenradius (R₅) und einem Innenradius (R₄) auf der lateralen Seite der Tibiaplattform (3) stattfindet.

10. Knieprothese nach Anspruch 9, **dadurch gekennzeichnet, dass** die Tibiaplattform symmetrisch zu ihrer Mittelebene (14) ausgeführt ist, und dass für jede kreisbogenförmige Nut (22) wahlweise ein verankerbarer Kunststoffkörper (12) oder ein bewegliches Meniskusteil (7) aus Kunststoff zur Verfügung steht.

## Claims

1. Knee prosthesis comprising a medial condyle (1), the running surface (2) of which and the counter-surface (A) of which that lies on a tibia platform (3) are sections of spherical surfaces with a first radius (Ri), the center (M₁') of which forms an axis (4) in its vertical projection to a first point (Mi) on the counter-surface (A) with a lateral condyle (5) being pivotable about the axis (4), wherein the running surface (6) of the lateral condyle (5) and the counter-surface (B) thereof are likewise sections of spherical surfaces with a second radius (R₂) with center (M₂'), the vertical projection of which onto the counter-surface (B) corresponds to a second point (M₂); and wherein the counter-surface (B) belongs to an artificial meniscus part (7) which can be displaced on a plane of the tibia platform and which, guided on a circular arc, is pivotable with the second point (M₂) about the first point (M₁) at a distance (R₃) which corresponds to the distance (a) of the centers (M1', M₂'), **characterized in that** the tibia platform (3) is made symmetrical with respect to the its sagittal midplane (14) in such a manner that each side is selectively suitable for the reception of a firmly anchorable plastic body (12) or of a movable meniscus part (7) in order to produce right and left tibia joint halves with one tibia platform (3).

2. Knee prosthesis in accordance with claim 1, **characterized in that**, when the meniscus part is held firmly, the tibia platform (3) is pivotable from a middle position, in which the points (M₁, M₁'; M₂, M₂') lie in a transverse plane (9) of the tibia platform (3), by an angle α₁ ≥ 0 towards the posterior and by an angle α₂ > α₁ towards the anterior about the axis (4 = M₁, M₁').

3. Knee prosthesis in accordance with claim 2, **characterized in that** the angle α₂ lies between 5° and 20°.

4. Knee prosthesis in accordance with any one of the claims 1 to 3, **characterized in that** the guiding of the meniscus part (7) takes place through a circular-arc-shaped bulge (10) of the tibia platform (3) on the inner side of the lateral half of the tibia platform (3) which engages into a groove (11) of the meniscus part (7).

5. Knee prosthesis in accordance with any one of the claims 1 to 4, **characterized in that** the counter-surface A is formed of a plastic body (12) which is secured on the tibia platform (3); and **in that** the meniscus part (7) consists of plastic.

6. Knee prosthesis in accordance with any one of the claims 1 to 5, **characterized in that** a first or second radius (R₁, R₂) of one condyle (1, 5) is smaller than the second or first radius (R₂, R₁) of the other condyle (5, 1) by a specific amount; and **in that** the counter-surface (A, B) belonging to this smaller condyle projects further upwardly by this specific amount.

7. Knee prosthesis in accordance with claim 4, **characterized in that** the bulge (10) with its outer guiding surface corresponds to a radius (R₅) to the first point (M₁); and **in that** the radius (R₅) corresponds to an amount between 25 mm and 50 mm.

8. Knee prosthesis in accordance with any one of the claims 2 to 7, **characterized in that** the sum of the angles α₁ and α₂ lies between 12° and 18°.

9. Knee prosthesis in accordance with any one of the claims 1 to 3, **characterized in that** the guiding of the meniscus part (7) takes place through a circular arc-shaped groove (22) with an outer radius (R₅) and an inner radius (R₄) on the lateral side of the tibia platform (3).

10. Knee prosthesis in accordance with claim 9, **characterized in that** the tibia platform is made symmetrical with respect to its midplane (14); and **in that** an anchorable plastic body (12) or a movable meniscus part (7) of plastic is selectively available for each circular arc-shaped groove (22).

## Revendications

1. Prothèse de genou avec un condyle interne (1) dont la surface de glissement (2) et la surface antagoniste (A) située sur une plate-forme de tibia (3) sont des sections de surfaces sphériques avec un premier rayon (R1), dont le centre (M1') qui constitue, avec le point (M1) qui en est la projection verticale sur la surface antagoniste (A), un axe (4), autour duquel un condyle latéral (5) pivote, moyennant quoi le condyle latéral (5) a une surface de glissement (6) et une surface antagoniste (B) qui sont également des sections de surfaces sphériques avec un deuxième rayon (R2) et un centre (M2'), dont la projection verticale sur la surface antagoniste (B) correspond à un deuxième point (M2), et moyennant quoi la surface antagoniste (B) appartient à une partie de ménisque artificiel (7), mobile sur le plan de la plate-forme du tibia, qui pivote, avec un guidage, sur un arc de cercle avec le deuxième point (M2) autour du premier point (M1) à une distance (R3) qui correspond à la distance « a » entre les centres (M1', M2'), **caractérisée en ce que** la plate-forme du tibia (3) est symétrique par rapport à son plan sagittal (14) de telle sorte que chaque côté, au choix, puisse être utilisé pour le logement d'un corps en matière plastique (12) ancré solidement ou d'une partie mobile de ménisque (7), afin de réaliser des moitiés droite et gauche d'articulation de tibia avec une plate-forme de tibia (3).

2. Prothèse de genou selon la revendication 1, **caractérisée en ce que**, lorsque la partie de ménisque est maintenue immobile, la plate-forme du tibia (3) pivote à partir d'une position centrale dans laquelle les points (M1, M1' ; M2, M2') se trouvent dans un plan transversal (9) de la plate-forme du tibia (3) , autour de l'axe (4) (M1, M1') avec un angle α1 ≥ 0 vers le côté postérieur et avec un angle α2 ≥ α1 vers le côté antérieur.

3. Prothèse de genou selon la revendication 2, **caractérisée en ce que** l'angle α2 est compris entre 5° et 20°.

4. Prothèse de genou selon l'une des revendications 1 à 3, **caractérisée en ce que** le guidage de la partie de ménisque (7) est réalisé à l'aide d'un bourrelet (10) en arc de cercle sur la plate-forme du tibia (3), du côté intérieur de la moitié latérale de la plate-forme du tibia (3) qui s'emboîte dans une rainure (11) de la partie de ménisque (7).

5. Prothèse de genou selon l'une des revendications 1 à 4, **caractérisée en ce que** la surface antagoniste (A) est constituée d'un corps en matière plastique (12) fixé sur la plate-forme du tibia (3) et **en ce que** la partie de ménisque (7) est en matière plastique.

6. Prothèse de genou selon l'une des revendications 1 à 5, **caractérisée en ce qu'**un rayon (R1, R2) d'un condyle (1, 5) est inférieur d'une certaine valeur au rayon (R2, R1) de l'autre condyle (5, 1) et **en ce que** la surface antagoniste (A, B) correspondant à ce condyle plus petit dépasse plus loin de cette valeur.

7. Prothèse de genou selon la revendication 4, **caractérisée en ce que** le bourrelet (10) correspond, avec sa surface de guidage externe à un rayon (R5) autour du premier point M1 et **en ce que** le rayon (R5) a une valeur comprise entre 25 mm et 50 mm.

8. Prothèse de genou selon l'une des revendications 2 à 7, **caractérisée en ce que** la somme des angles α1 et α2 est comprise entre 12° et 18°.

9. Prothèse de genou selon l'une des revendications 1 à 3, **caractérisée en ce que** le guidage de la partie de ménisque (7) est réalisé à l'aide d'une rainure (22) en arc de cercle avec un rayon extérieur (R5) et un rayon intérieur (R4) sur le côté latéral de la plate-forme du tibia (3).

10. Prothèse de genou selon la revendication 9, **caractérisée en ce que** la plate-forme du tibia est symétrique par rapport à son plan médian (14 et **en ce que**, dans chaque rainure (22) en arc de cercle, se trouve soit un corps en matière plastique (12) soit une partie mobile de ménisque (7) en matière plastique.
